# EUROPEAN PATENT SPECIFICATION

(11) **EP 4 162 053 B1**
(45) Date of publication and mention of the grant of the patent: **28.10.2026**
(21) Application number: 21818924.9
(22) Date of filing: 04.06.2021
(51) Int. Cl.: G06N 3/09, G06N 3/0475, G16B 40/20, G16B 30/20, C12N 15/62, G06N 3/045, G06N 3/02, G06N 3/08, G06N 3/082, G06N 3/044

(54) **SYSTEMS AND METHODS FOR GENERATING A SIGNAL PEPTIDE AMINO ACID SEQUENCE USING DEEP LEARNING**
SYSTEME UND VERFAHREN ZUR ERZEUGUNG EINER SIGNALPEPTIDAMINOSÄURESEQUENZ UNTER VERWENDUNG VON TIEFENLERNEN
SYSTÈMES ET PROCÉDÉS POUR GÉNÉRER UNE SÉQUENCE D'ACIDES AMINÉS DE PEPTIDE SIGNAL À L'AIDE D'UN APPRENTISSAGE PROFOND

(30) Priority: 04.06.2020 US 202063034802 P
(43) Date of publication of application: 12.04.2023
(73) Proprietor: California Institute of Technology, Pasadena, CA 91125 (US); BASF SE, 67056 Ludwigshafen am Rhein (DE)
(72) Inventor: LISZKA, Michael, San Diego, California 92121 (US); WU, Zachary, Pasadena, California 91125 (US); YANG, Kevin, Pasadena, California 91125 (US)
(74) Representative: Maiwald GmbH
(86) International application number: PCT/US2021/035990
(87) International publication number: WO 2021/248060

(56) References cited:
- WO-A2-2008/110282
- BARASH S ET AL: "Human secretory signal peptide description by hidden Markov model and generation of a strong artificial signal peptide for secreted protein expression", BIOCHEMICAL AND BIOPHYSICAL RESEARCH COMMUNICATIONS, ELSEVIER, AMSTERDAM NL, vol. 294, no. 4, 21 June 2002 (2002-06-21), pages 835 - 842, XP002292654, ISSN: 0006-291X, DOI: 10.1016/S0006-291X(02)00566-1
- WREDE P ET AL: "Peptide design aided by neural networks: biological activity of rtificial signal peptidase I cleavage sites", BIOCHEMISTRY, AMERICAN CHEMICAL SOCIETY, vol. 37, no. 11, 17 March 1998 (1998-03-17), pages 3588 - 3593, XP002127397, ISSN: 0006-2960, DOI: 10.1021/BI9726032
- WU ZACHARY ET AL: "Signal Peptides Generated by Attention-Based Neural Networks", vol. 9, no. 8, 21 August 2020 (2020-08-21), Washington DC ,USA, pages 2154 - 2161, XP055880739, ISSN: 2161-5063, Retrieved from the Internet <URL:https://pubs.acs.org/doi/pdf/10.1021/acssynbio.0c00219> DOI: 10.1021/acssynbio.0c00219
- FU GANG ET AL: "Systematic Screening of Optimal Signal Peptides for Secretory Production of Heterologous Proteins in Bacillus subtilis", vol. 66, no. 50, 22 November 2018 (2018-11-22), US, pages 13141 - 13151, XP055948315, ISSN: 0021-8561, Retrieved from the Internet <URL:http://pubs.acs.org/doi/pdf/10.1021/acs.jafc.8b04183> DOI: 10.1021/acs.jafc.8b04183
- FREUDL ROLAND: "Signal peptides for recombinant protein secretion in bacterial expression systems", vol. 17, no. 1, 1 December 2018 (2018-12-01), pages 52, XP055914048, Retrieved from the Internet <URL:https://link.springer.com/content/pdf/10.1186/s12934-018-0901-3.pdf> DOI: 10.1186/s12934-018-0901-3
- SAVOJARDO CASTRENSE ET AL: "DeepSig: deep learning improves signal peptide detection in proteins", vol. 34, no. 10, 15 May 2018 (2018-05-15), GB, pages 1690 - 1696, XP055880736, ISSN: 1367-4803, Retrieved from the Internet <URL:https://watermark.silverchair.com/btx818.pdf?token=AQECAHi208BE49Ooan9kkhW_Ercy7Dm3ZL_9Cf3qfKAc485ysgAAAvgwggL0BgkqhkiG9w0BBwagggLlMIIC4QIBADCCAtoGCSqGSIb3DQEHATAeBglghkgBZQMEAS4wEQQMmXNCrONyKSlYaLSaAgEQgIICq8D5aJl7TJJzg0DgWcvuDwYs0bFqyf92R6aDo-m9ZaMQjpA2Spnu3Ho4xRvUFA3-IZsGjK2f8pOq_pYSxqCmcz34S9aKz> DOI: 10.1093/bioinformatics/btx818
- SAVOJARDO CASTRENSE, MARTELLI PIER LUIGI, FARISELLI PIERO, CASADIO RITA: "DeepSig: deep learning improves signal peptide detection in proteins", BIOINFORMATICS, OXFORD UNIVERSITY PRESS , SURREY, GB, vol. 34, no. 10, 15 May 2018 (2018-05-15), GB , pages 1690 - 1696, XP055880736, ISSN: 1367-4803, DOI: 10.1093/bioinformatics/btx818
- JEIRANIKHAMENEH MEISAM, MOSHIRI FARZANEH, FALASAFI SOHEIL KEYHAN, ZOMORODIPOUR ALIREZA: "Designing Signal Peptides for Efficient Periplasmic Expression of Human Growth Hormone in Escherichia coli", JOURNAL OF MICROBIOLOGY AND BIOTECHNOLOGY, vol. 27, no. 11, 28 November 2017 (2017-11-28), pages 1999 - 2009, XP055880737, ISSN: 1017-7825, DOI: 10.4014/jmb.1703.03080
- WU ZACHARY, YANG KEVIN K., LISZKA MICHAEL J., LEE ALYCIA, BATZILLA ALINA, WERNICK DAVID, WEINER DAVID P., ARNOLD FRANCES H.: "Signal Peptides Generated by Attention-Based Neural Networks", ACS SYNTHETIC BIOLOGY, AMERICAN CHEMICAL SOCIETY, WASHINGTON DC ,USA, vol. 9, no. 8, 21 August 2020 (2020-08-21), Washington DC ,USA , pages 2154 - 2161, XP055880739, ISSN: 2161-5063, DOI: 10.1021/acssynbio.0c00219

## Description

### FIELD OF TECHNOLOGY

The present disclosure relates to the field of biotechnology, and, more specifically, to systems and methods for generating a signal peptide (SP) amino acid sequence using deep learning.

### BACKGROUND

For cells to function, proteins must be targeted to their proper locations. To direct a protein, organisms encode instructions in a leading short peptide sequence (typically 15-30 amino acids) called a signal peptide (SP). SPs have been engineered for a variety of industrial and therapeutic purposes, including increased export for recombinant protein production and increasing the therapeutic levels of proteins secreted from industrial production hosts.

Due to the utility and ubiquity of protein secretion pathways, a significant amount of work has been invested in identifying SPs in natural protein sequences. Conventionally, machine learning has been used to analyze an input enzyme sequence and classify the portion of the sequence that is the SP. While this allows for the identification of SP sequences, generating a SP sequence itself and validating the functionality of the generated SP sequence in vivo has yet to be performed.

Given a desired protein to target for secretion, there is no universally-optimal directing SP and there is no reliable method for generating a SP with measurable activity. Instead, libraries of naturally-occurring SP sequences from the host organism or phylogenetically-related organisms are tested for each new protein secretion target. While researchers have attempted to generalize the understanding of SP-protein pairs by developing general SP design guidelines, those guidelines are heuristics at best and are limited to modifying existing SPs, not designing new ones. For instance, "Human secretory signal peptide description by hidden Markov model and generation of a strong artificial signal peptide for secreted protein expression" by BARASH S et al. in BIOCHEMICAL AND BIOPHYSICAL RESEARCH COMMUNICATIONS vol. 294, no. 4, pages 835-842 discloses a trained Hidden-Markov model for generating signal peptide sequences.

### SUMMARY OF VARIOUS ASPECTS OF THE INVENTION

To address these and other needs, aspects of the present disclosure describe methods and systems for generating a signal peptide (SP) amino acid sequence using deep learning. In one exemplary aspect, such methods may train a deep machine learning model to generate functional SP sequences for protein sequences using a dataset that maps a plurality of output SP sequences to a plurality of corresponding input protein sequence. The method may thus, generate, via the trained deep machine learning model, an output SP sequence for an input protein sequence. In an exemplary aspect, the trained deep machine learning model may be configured to receive the input protein sequence, tokenize each amino acid of the input protein sequence to generate a sequence of tokens, map the sequence of tokens to a sequence of continuous representations via an encoder, and generate the output SP sequence based on the sequence of continuous representations via a decoder.

It should be noted that the aspects described herein may be implemented in a system comprising a hardware processor. Alternatively, such methods may be implemented using computer-executable instructions stored in a non-transitory computer readable medium.

The above simplified summary of exemplary aspects serves to provide a basic understanding of the present disclosure. This summary is not an extensive overview of all contemplated aspects, and is not intended to identify key or critical elements of all aspects nor delineate the scope of any or all aspects of the present disclosure. Its sole purpose is to present one or more aspects in a simplified form as a prelude to the more detailed description of the disclosure that follows. To the accomplishment of the foregoing, the one or more aspects of the present disclosure include the features described and exemplarily pointed out in the claims.

### BRIEF DESCRIPTION OF THE DRAWINGS

The accompanying drawings, which are incorporated into and constitute a part of this specification, illustrate one or more exemplary aspects of the present disclosure and, together with the detailed description, serve to explain their principles and implementations.
FIG. 1 is a block diagram illustrating a system for generating a SP amino acid sequence using deep learning, in accordance with aspects of the present disclosure.
FIG. 2 illustrates a flow diagram of an exemplary method for generating a SP amino acid sequence using deep learning, in accordance with aspects of the present disclosure.
FIG. 3 illustrates an example of a general-purpose computer system on which aspects of the present disclosure can be implemented.

### DETAILED DESCRIPTION

Exemplary aspects are described herein in the context of a system, method, and computer program product for generating a signal peptide (SP) amino acid sequence using deep learning. Those of ordinary skill in the art will realize that the following description is illustrative only and is not intended to be in any way limiting. Other aspects will readily suggest themselves to those skilled in the art having the benefit of this disclosure. Reference will now be made in detail to implementations of the examplary aspects as illustrated in the accompanying drawings. The same reference indicators will be used to the extent possible throughout the drawings and the following description to refer to the same or like items.

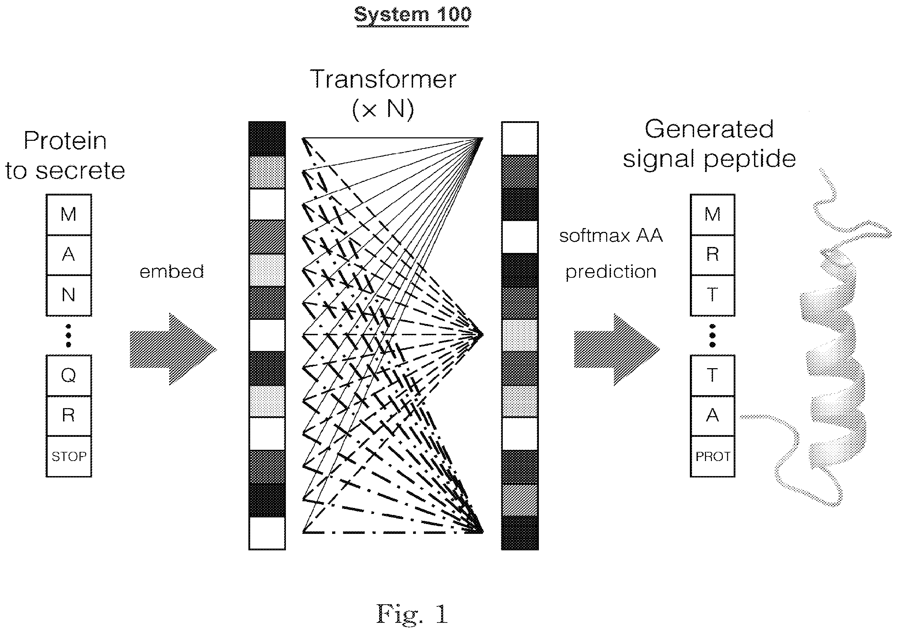

FIG. 1 is a block diagram illustrating system 100 for generating a SP amino acid sequence using deep learning, in accordance with aspects of the present disclosure. System 100 depicts an exemplary deep machine learning model utilized in the present disclosure. In some aspects, the deep machine learning model is an artificial neural network with an encoder-decoder architecture (henceforth, a "transformer"). A transformer is designed to handle ordered sequences of data, such as natural language, for various tasks such as translation. Ultimately, a transformer receives an input sequence and generates an output sequence. For example, in the context of natural language processing the input sequence may be a sentence. Because a transformer does not require that the input sequence be processed in order, the transformer does not need to process the beginning of a sentence before it processes the end. This allows for parallelization and greater efficiency when compared to counterpart neural networks such as recurrent neural networks. While the present disclosure focuses on transformers having an encoder-decoder architecture, it is understood that in alternative aspects, the methods described herein may instead use an artificial neural network which implements a singular encoder or decoder architecture rather than a paired encoder-decoder architecture. Such architectures may be used to carry out any of the methods described herein.

In some aspects, the dataset used to train the neural network used by the systems described herein may comprise a map which associates a plurality of known output SP sequences to a plurality of corresponding known input protein sequence. For example, the plurality of known input protein sequences used for training may include SEQ ID NO: 1, which is known to have the output SP sequence represented by SEQ ID NO: 2. Another known input protein sequence may be SEQ ID NO: 3, which in turn corresponds to the known output SP sequence represented by SEQ ID NO: 4. SEQ ID NOs: 1-4 are shown on Table 1 below:

**Table 1: Exemplary known input protein sequences and known output SP sequences.**

| | |
|---|---|
| SEQ ID NO: 1 | AERQPLKIPPIIDVGRGRPVRLDLRPAQTQFDKGKLVDVWGVNGQYLAPTV RVKSDDFVKLTYVNNLPQTVTMNIQGLLAPTDMIGSIHRKLEAKSSWSPIISI HQPACTCWYHADTMLNSAFQIYRGLAGMWIIEDEQSKKANLPNKYGVNDIP LILQDQQLNKQGVQVLDANQKQFFGKRLFVNGQESAYHQVARGWVRLRIV NASLSRPYQLRLDNDQPLHLIATGVGMLAEPVPLESITLAPSERVEVLVELNE GKTVSLISGQKRDIFYQAKNLFSDDNELTDNVILELRPEGMAAVFSNKPSLPP FATEDFQLKIAEERRLIIRPFDRLINQKRFDPKRIDFNVKQGNVERWYITSDEA VGFTLQGAKFLIETRNRQRLPHKQPAWHDTVWLEKNQEVTLLVRFDHQAS AQLPFTFGVSDFMLRDRGAMGQFIVTE |
| SEQ ID NO: 2 | MMNLTRRQLLTRSAVAATMFSAPKTLWA |
| SEQ ID NO: 3 | ERIKDLTTIQGVRSNQLIGYGLVVGLDGTGDQTTQTPFTVQSIVSMMQQMGI NLPSGTNLQLRNVAAVMVTGNLPPFAQPGQPMDVTVSSMGNARSLRGGTL LMTPLKGADNQVYAMAQGNLVIGGAGAGASGTSTQINHLGAGRISAGAIVE RAVPSQLTETSTIRLELKEADFSTASMVVDAINKRFGNGTATPLDGRVIQVQP PMDINRIAFIGNLENLDVKPSQGPAKVILNARTGSVVMNQAVTLDDCAISHG NLSVVINTAPAISQPGPFSGGQTVATQVSQVEINKEPGQVIKLDKGTSLADVV KALNAIGATPQDLVAILQAMKAAGSLRADLEII |
| SEQ ID NO: 4 | MTLTRPLALISALAALILALPADA |

Table 1 illustrates two exemplary pairs of known input protein sequences and their respective known output SP sequences. It is understood that the dataset used to train the neural network implemented by the systems described herein may include, e.g., hundreds or thousands of such pairs. A set of known protein sequences, and their respective known SP sequences, can be generated using publicly-accessible databases (e.g., the NCBI or UniProt databases) or proprietary sequencing data. For example, many publicly-accessible databases include annotated polypeptide sequences which identify the start and end position of experimentally validated SPs. In some aspects, the known SP for a given known input protein sequence may be a predicted SP (e.g., identified using a tool such as the SignalP server described in Armenteros, J. et al., "SignalP 5.0 improves signal peptide predictions using deep neural networks." Nature Biotechnology 37.4 (2019): 420-423.

In some aspects, the neural network used by the systems described herein leverages an attention mechanism, which weights different positions over a given input protein sequence in order to determine a representation of that sequence. The transformer architecture is applied to SP prediction by treating each of the amino acids as a token. In some aspects, the transformer comprises two components: an encoder and decoder. In other aspects, the transformer may comprise a chain of encoders and a chain of decoders. The transformer's encoder maps an input sequence of tokens (e.g., the amino acids of a known protein sequence) to a sequence of continuous representations. The sequence of continuous representations is a machine interpretation of the input tokens that relates the positions in each input protein sequence with the positions in each output SP sequence. Given these representations, the decoder may then generate an output sequence (the SP amino acids), one token at a time. Each step in this generation process depends on the generated sequence elements preceding the current step and continues until a special <END OF SP> token is generated. FIG. 1 illustrates this modeling scheme.

In some aspects, the transformer is configured to have multiple layers (e.g., 2 - 10 layers) and/or hidden dimensions (e.g., 128 - 2,056 hidden dimensions). For example, the transformer may have 5 layers and a hidden dimension of 550. Each layer may comprise multiple attention heads (e.g., 4 - 10 attention heads). For example, each layer may comprise 6 attention heads. Training may be performed, for multiple epochs (e.g., 50 - 200 epochs) with a user-selected dropout rate (e.g., in the range of 0.1 - 0.8). For example, training may be performed for 100 epochs with a dropout rate of 0.1 in each attention head and after each position-wise feed-forward layer. In some aspects, periodic positional encodings and an optimizer may be used in the transformer. For example, the Adam or Lamb optimizer may be used. In some aspects, the learning rate schedule may include a warmup period followed by exponential or sinusoidal decay. For example, the learning rate can be increased linearly for a first set of batches (e.g., the first 12,500 batches) from 0 to 1e-4 and then decayed by n_steps^{-0.03} after the linear warmup. It should be noted that one skilled in the art may adjust these numerical values to potentially improve the accuracy of functional SP sequence generation.

**In** some aspects, various sub-sequences of the input protein sequences may be used as source sequences in order to augment the training dataset, to diminish the effect of choosing one specific length cutoff, and to make the model more robust. For example, the systems may be configured such that for input proteins of length L < 105, the model receives the first L - 10, L - 5, and L residues as training inputs. The system may also be configured, in some aspects, such that for mature proteins of L >= 105, the model receives the first 95, 100, and 105 amino residues as training inputs. It should be noted that the specific cutoff lengths and amino residues described above may be adjusted to improve the accuracy of functional SP sequence generation.

In some aspects, in addition to training on a full dataset, the transformer may be trained on subsets of the full dataset. For example, subsets may remove sequences with ≥75%, ≥90%, ≥95%, or ≥99% sequence identity to a selected protein or to a plurality of proteins (e.g., a class of enzymes) in order to test the model's ability to generalize to distant protein sequences. Accordingly, the transformer may be trained on a full dataset and truncated versions of the full dataset.

Given a trained deep machine learning model that predicts sequence probabilities, there are various approaches by which protein sequences can be generated. In some aspects, a beam search is applied. A beam search is a heuristic search algorithm that traverses a graph by expanding the most probable node in a limited set. In the present disclosure, the beam search generates a sequence by taking the most probable amino acid additions from the N-terminus (i.e., the start of a protein or polypeptide referring to the free amine group located at the end of a polypeptide). In some aspects, a mixed input beam search may be used over the decoder to generate a "generalist" SP, which has the highest probability of functioning across multiple input protein sequences. The beam size for the mixed input beam search may be 5. In traditional beam search, the size of the beam refers to the number of unique hypotheses with highest predicted probability for a specific input that are tracked at each generation step. In contrast, the mixed input beam search generates hypotheses for multiple inputs (rather than one), keeping the sequences with highest predicted probabilities.

In some aspects, the trained deep machine learning model may output an SP sequence for an input protein sequence. The output SP sequence may then be queried for novelty (i.e., whether the sequence exists in a database of known functional SP sequences). In some aspects, in response to determining that the output SP sequence is novel, the output SP sequence may be tested for functionality.

In some aspects, the systems described herein may be used to generate a construct that merges the generated output SP sequence and the input protein sequence. Such constructs comprise the sequence of an SP-protein pair whose functionality may be evaluated by experimentally verifying whether the protein associated with the input protein sequence is localized extracellularly (e.g., secreted) and acquires a native three-dimensional structure that remains biologically functional when a signal peptide corresponding to the output SP sequence serves as an amino terminus of the protein. This verification may be performed by expressing the construct (i.e., a generated SP-protein pair) in a host cell, e.g., a gram-positive bacterial host such as *Bacillus subtilis,* which is useful for secretion of industrial enzymes.

In response to determining that the construct is functional, the SP-protein pair may be deemed functional. In response to determining that the construct is not functional, the deep machine learning model may be further trained to improve accuracy of SP generation.

As noted above, the deep machine learning model may be trained using inputs that comprise a plurality of known SP-protein pairs (e.g., a set of known protein sequences and their respective known SP sequences). Accordingly, the deep machine learning model learns the characteristics of how SP sequences are positioned relative to their respective protein sequences. As such, in some aspects the present systems (after training with a sufficient dataset) may be used to identify the SP in any arbitrary SP-protein pair. A focus of identification is to determine length and positioning of the SP sequence. In contrast, when the present systems are used to generate an SP sequence for an arbitrary protein sequence selected as an input, the model must typically account for the structural and sequential parameters of the SP and/or the input protein.

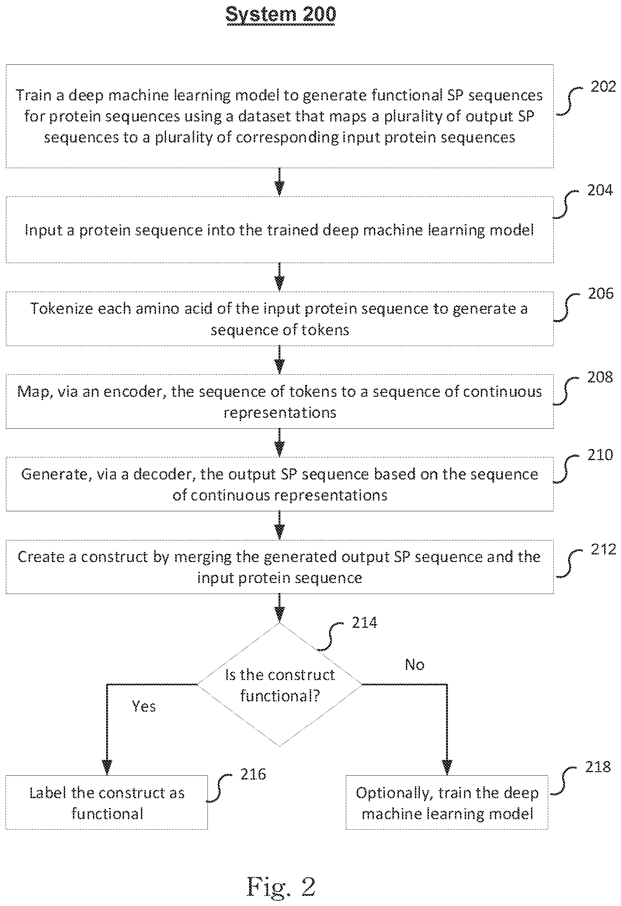

FIG. 2 illustrates a flow diagram of an exemplary method 200 for generating an SP amino acid sequence using deep learning, in accordance with aspects of the present disclosure. At 202, method 200 trains a deep machine learning model to generate functional SP sequences for protein sequences using a dataset that maps a plurality of output SP sequences to a plurality of corresponding input protein sequences. For example, the deep machine learning model may have a transformer encoder-decoder architecture depicted in system 100.

At 204, method 200 inputs a protein sequence in the trained deep machine learning model. For example, the input protein sequence may have the following sequence:

“DGLNGTMMQYYEWHLENDGQHWNRLHDDAAALSDAGITAIWIPPAYKGNSQADVGYGAYD
LYDLGEFNQKGTVRTKYGTKAQLERAIGSLKSNDINVYGD” (SEQ ID NO: 5).

At 206, the trained deep machine learning model tokenizes each amino acid of the input protein sequence to generate a sequence of tokens. In some aspects, the tokens may be individual amino acids of the input protein sequence (e.g., SEQ ID NO: 5) listed above.

At 208, the trained deep machine learning model maps, via an encoder, the sequence of tokens to a sequence of continuous representations. The continuous representations may be machine interpretations of the positions of tokens relative to each other.

At 210, the trained deep machine learning model generates, via a decoder, the output SP sequence based on the sequence of continuous representations. For example, the output SP sequence may be "MKLLTSFVLIGALAFA" (SEQ ID NO: 6).

At 212, method 200 creates a construct by merging the generated output SP sequence and the input protein sequence. The construct in the overarching example may thus be:

“MKLLTSFVLIGALAFADGLNGTMMQYYEWHLENDGQHWNRLHDDAALSDAGITAI
WIPPAYKGNSQADVGYGAYDLYDLGEFNQKGTVRTKYGTKAQLERAIGSLKSNDINVY
GD” (SEQ ID NO: 7).

At 214, method 200 may comprise determining whether the construct (SEQ ID NO: 7) is in fact functional. More specifically, method 200 determines whether the protein associated with the input protein sequence (SEQ ID NO: 5) is localized extracellularly and acquires a native three-dimensional structure that is biologically functional when a signal peptide corresponding to the output SP sequence "MKLLTSFVLIGALAFA" (SEQ ID NO: 6) serves as an amino terminus of the protein.

In response to determining that the construct is functional, at 216, method 200 labels the construct as functional. However, in response to determining that the construct is not functional, at 218, method 200 may further train the deep machine learning model. In this particular example, the output SP sequence "MKLLTSFVLIGALAFA" (SEQ ID NO: 6) produces a functional construct.

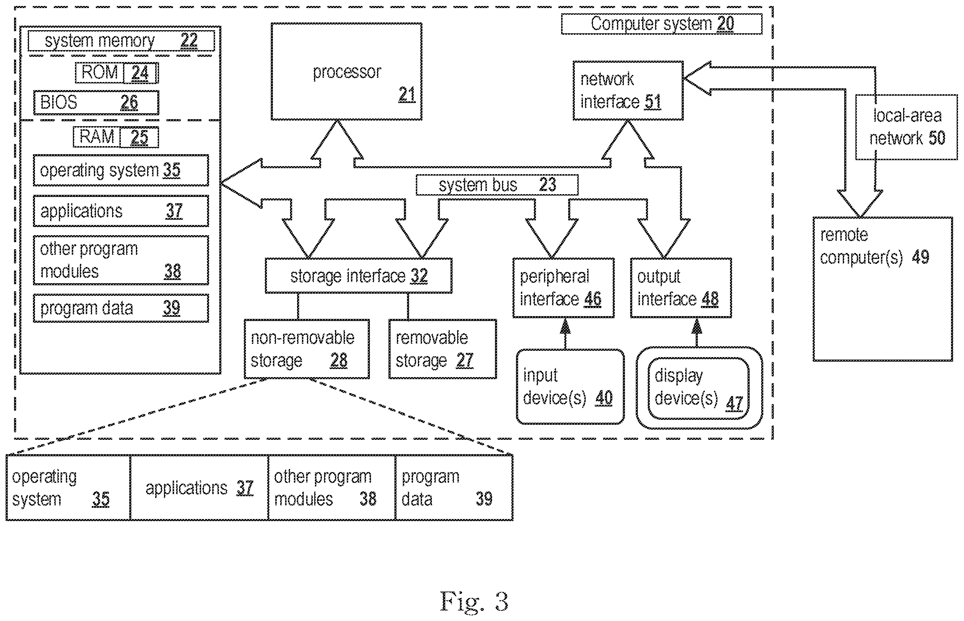

FIG. 3 is a block diagram illustrating a computer system 20 on which aspects of systems and methods for generating SP amino acid sequences using deep learning may be implemented in accordance with an exemplary aspect. The computer system 20 can be in the form of multiple computing devices, or in the form of a single computing device, for example, a desktop computer, a notebook computer, a laptop computer, a mobile computing device, a smart phone, a tablet computer, a server, a mainframe, an embedded device, and other forms of computing devices.

As shown, the computer system 20 includes a central processing unit (CPU) 21, a graphics processing unit (GPU), a system memory 22, and a system bus 23 connecting the various system components, including the memory associated with the central processing unit 21. The system bus 23 may comprise a bus memory or bus memory controller, a peripheral bus, and a local bus that is able to interact with any other bus architecture. Examples of the buses may include PCI, ISA, PCI-Express, HyperTransport^{™}, InfiniBand^{™}, Serial ATA, I²C, and other suitable interconnects. The central processing unit 21 (also referred to as a processor) can include a single or multiple sets of processors having single or multiple cores. The processor 21 may execute one or more computer-executable code implementing the techniques of the present disclosure. For example, any of commands/steps discussed in FIGS. 1-2 may be performed by processor 21. The system memory 22 may be any memory for storing data used herein and/or computer programs that are executable by the processor 21. The system memory 22 may include volatile memory such as a random access memory (RAM) 25 and non-volatile memory such as a read only memory (ROM) 24, flash memory, etc., or any combination thereof. The basic input/output system (BIOS) 26 may store the basic procedures for transfer of information between elements of the computer system 20, such as those at the time of loading the operating system with the use of the ROM 24.

The computer system 20 may include one or more storage devices such as one or more removable storage devices 27, one or more non-removable storage devices 28, or a combination thereof. The one or more removable storage devices 27 and non-removable storage devices 28 are connected to the system bus 23 via a storage interface 32. In an aspect, the storage devices and the corresponding computer-readable storage media are power-independent modules for the storage of computer instructions, data structures, program modules, and other data of the computer system 20. The system memory 22, removable storage devices 27, and non-removable storage devices 28 may use a variety of computer-readable storage media. Examples of computer-readable storage media include machine memory such as cache, SRAM, DRAM, zero capacitor RAM, twin transistor RAM, eDRAM, EDO RAM, DDR RAM, EEPROM, NRAM, RRAM, SONOS, PRAM; flash memory or other memory technology such as in solid state drives (SSDs) or flash drives; magnetic cassettes, magnetic tape, and magnetic disk storage such as in hard disk drives or floppy disks; optical storage such as in compact disks (CD-ROM) or digital versatile disks (DVDs); and any other medium which may be used to store the desired data and which can be accessed by the computer system 20.

The system memory 22, removable storage devices 27, and non-removable storage devices 28 of the computer system 20 may be used to store an operating system 35, additional program applications 37, other program modules 38, and program data 39. The computer system 20 may include a peripheral interface 46 for communicating data from input devices 40, such as a keyboard, mouse, stylus, game controller, voice input device, touch input device, or other peripheral devices, such as a printer or scanner via one or more I/O ports, such as a serial port, a parallel port, a universal serial bus (USB), or other peripheral interface. A display device 47 such as one or more monitors, projectors, or integrated display, may also be connected to the system bus 23 across an output interface 48, such as a video adapter. In addition to the display devices 47, the computer system 20 may be equipped with other peripheral output devices (not shown), such as loudspeakers and other audiovisual devices.

The computer system 20 may operate in a network environment, using a network connection to one or more remote computers 49. The remote computer (or computers) 49 may be local computer workstations or servers comprising most or all of the aforementioned elements in describing the nature of a computer system 20. Other devices may also be present in the computer network, such as, but not limited to, routers, network stations, peer devices or other network nodes. The computer system 20 may include one or more network interfaces 51 or network adapters for communicating with the remote computers 49 via one or more networks such as a local-area computer network (LAN) 50, a wide-area computer network (WAN), an intranet, and the Internet. Examples of the network interface 51 may include an Ethernet interface, a Frame Relay interface, SONET interface, and wireless interfaces.

Aspects of the present disclosure may be a system, a method, and/or a computer program product. The computer program product may include a computer readable storage medium (or media) having computer readable program instructions thereon for causing a processor to carry out aspects of the present disclosure.

The computer readable storage medium can be a tangible device that can retain and store program code in the form of instructions or data structures that can be accessed by a processor of a computing device, such as the computing system 20. The computer readable storage medium may be an electronic storage device, a magnetic storage device, an optical storage device, an electromagnetic storage device, a semiconductor storage device, or any suitable combination thereof. By way of example, such computer-readable storage medium can comprise a random access memory (RAM), a read-only memory (ROM), EEPROM, a portable compact disc read-only memory (CD-ROM), a digital versatile disk (DVD), flash memory, a hard disk, a portable computer diskette, a memory stick, a floppy disk, or even a mechanically encoded device such as punch-cards or raised structures in a groove having instructions recorded thereon. As used herein, a computer readable storage medium is not to be construed as being transitory signals per se, such as radio waves or other freely propagating electromagnetic waves, electromagnetic waves propagating through a waveguide or transmission media, or electrical signals transmitted through a wire.

Computer readable program instructions described herein can be downloaded to respective computing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network interface in each computing device receives computer readable program instructions from the network and forwards the computer readable program instructions for storage in a computer readable storage medium within the respective computing device.

Computer readable program instructions for carrying out operations of the present disclosure may be assembly instructions, instruction-set-architecture (ISA) instructions, machine instructions, machine dependent instructions, microcode, firmware instructions, state-setting data, or either source code or object code written in any combination of one or more programming languages, including an object oriented programming language, and conventional procedural programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a LAN or WAN, or the connection may be made to an external computer (for example, through the Internet). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present disclosure.

In various aspects, the systems and methods described in the present disclosure can be addressed in terms of modules. The term "module" as used herein refers to a real-world device, component, or arrangement of components implemented using hardware, such as by an application specific integrated circuit (ASIC) or FPGA, for example, or as a combination of hardware and software, such as by a microprocessor system and a set of instructions to implement the module's functionality, which (while being executed) transform the microprocessor system into a special-purpose device. A module may also be implemented as a combination of the two, with certain functions facilitated by hardware alone, and other functions facilitated by a combination of hardware and software. In certain implementations, at least a portion, and in some cases, all, of a module may be executed on the processor of a computer system. Accordingly, each module may be realized in a variety of suitable configurations, and should not be limited to any particular implementation exemplified herein.

In the interest of clarity, not all of the routine features of the aspects are disclosed herein. It would be appreciated that in the development of any actual implementation of the present disclosure, numerous implementation-specific decisions must be made in order to achieve the developer's specific goals, and these specific goals will vary for different implementations and different developers. It is understood that such a development effort might be complex and time-consuming, but would nevertheless be a routine undertaking of engineering for those of ordinary skill in the art, having the benefit of this disclosure.

Furthermore, it is to be understood that the phraseology or terminology used herein is for the purpose of description and not of restriction, such that the terminology or phraseology of the present specification is to be interpreted by the skilled in the art in light of the teachings and guidance presented herein, in combination with the knowledge of those skilled in the relevant art(s). Moreover, it is not intended for any term in the specification or claims to be ascribed an uncommon or special meaning unless explicitly set forth as such.

The various aspects disclosed herein encompass present and future known equivalents to the known modules referred to herein by way of illustration. Moreover, while aspects and applications have been shown and described, it would be apparent to those skilled in the art having the benefit of this disclosure that many more modifications than mentioned above are possible without departing from the inventive concepts disclosed herein.

### SEQUENCE LISTING

| **SEQ ID NO:** | **AMINO ACID SEQUENCE** |
|---|---|
| 1 | AERQPLKIPPIIDVGRGRPVRLDLRPAQTQFDKGKLVDVWGVNGQYL APTVRVKSDDFVKLTYVNNLPQTVTMNIQGLLAPTDMIGSIHRKLEA KSSWSPIISIHQPACTCWYHADTMLNSAFQIYRGLAGMWIIEDEQSKK ANLPNKYGVNDIPLILQDQQLNKQGVQVLDANQKQFFGKRLFVNGQ ESAYHQVARGWVRLRIVNASLSRPYQLRLDNDQPLHLIATGVGMLA EPVPLESITLAPSERVEVLVELNEGKTVSLISGQKRDIFYQAKNLFSDD NELTDNVILELRPEGMAAVFSNKPSLPPFATEDFQLKIAEERRLIIRPFD RLINQKRFDPKRIDFNVKQGNVERWYITSDEAVGFTLQGAKFLIETRN RQRLPHKQPAWHDTVWLEKNQEVTLLVRFDHQASAQLPFTFGVSDF MLRDRGAMGQFIVTE |
| 2 | MMNLTRRQLLTRSAVAATMF SAPKTLWA |
| 3 | ERIKDLTTIQGVRSNQLIGYGLVVGLDGTGDQTTQTPFTVQSIVSMMQ QMGINLPSGTNLQLRNVAAVMVTGNLPPFAQPGQPMDVTVSSMGNA RSLRGGTLLMTPLKGADNQVYAMAQGNLVIGGAGAGASGTSTQINH LGAGRISAGAIVERAVPSQLTETSTIRLELKEADFSTASMVVDAINKRF GNGTATPLDGRVIQVQPPMDINRIAFIGNLENLDVKPSQGPAKVILNA RTGSVVMNQAVTLDDCAISHGNLSVVINTAPAISQPGPFSGGQTVAT QVSQVEINKEPGQVIKLDKGTSLADVVKALNAIGATPQDLVAILQAM KAAGSLRADLEII |
| 4 | MTLTRPLALISALAALILALPADA |
| 5 | DGLNGTMMQYYEWHLENDGQHWNRLHDDAAALSDAGITAIWIPPA YKGNSQADVGYGAYDLYDLGEFNQKGTVRTKYGTKAQLERAIGSLK SNDINVYGD |
| 6 | MKLLTSFVLIGALAFA |
| 7 | MKLLTSFVLIGALAFADGLNGTMMQYYEWHLENDGQHWNRLHDDA AALSDAGITAIWIPPAYKGNSQADVGYGAYDLYDLGEFNQKGTVRT KYGTKAQLERAIGSLKSNDINVYGD |

## Claims

1. A computer-implemented method for generating a signal peptide (SP) amino acid sequence, comprising:
training a deep machine learning model to generate functional SP sequences for protein sequences using a dataset that maps a plurality of output SP sequences to a plurality of corresponding input protein sequences;
generating, via the trained deep machine learning model, an output SP sequence for an input protein sequence, wherein the trained deep machine learning model is configured to:
receive the input protein sequence;
tokenize each amino acid of the input protein sequence to generate a sequence of tokens;
map, via an encoder, the sequence of tokens to a sequence of continuous representations; and
generate, via a decoder, the output SP sequence based on the sequence of continuous representations.

2. The method of claim 1, further comprising:
creating a construct by merging the generated output SP sequence and the input protein sequence;
determining whether the construct is functional by verifying whether a protein corresponding to the input protein sequence
(1) is localized extracellularly and
(2) acquires a native three-dimensional structure that is biologically functional,
when a signal peptide corresponding to the output SP sequence serves as an amino terminus of the protein.

3. The method of claim 2, further comprising:
in response to determining that the construct is functional, labeling the construct as functional; and
in response to determining that the construct is non-functional, further training the deep machine learning model using the dataset, wherein each mapping in the dataset produces a functional construct.

4. The method of claim 2, wherein the verifying step comprises expressing a protein having the sequence of the construct in a gram-positive host cell and detecting whether the protein is secreted.

5. The method of claim 4, wherein the gram-positive host cell is a *Bacillus subtilis* cell.

6. The method of claim 1, wherein the deep machine learning model comprises an attention mechanism that incorporates a context of a respective amino acid in a given input sequence to generate an output sequence.

7. A system for generating a signal peptide (SP) amino acid sequence, comprising:
a hardware processor configured to:
train a deep machine learning model to generate functional SP sequences for protein sequences using a dataset that maps a plurality of output SP sequences to a plurality of corresponding input protein sequences;
generate, via the trained deep machine learning model, an output SP sequence for an input protein sequence, wherein the trained deep machine learning model is configured to:
receive the input protein sequence;
tokenize each amino acid of the input protein sequence to generate a sequence of tokens;
map, via an encoder, the sequence of tokens to a sequence of continuous representations; and
generate, via a decoder, the output SP sequence based on the sequence of continuous representations.

8. The system of claim 7, wherein the hardware processor is further configured to:
create a construct by merging the generated output SP sequence and the input protein sequence;
receive an indication of whether the construct is functional;
in response to determining that the construct is functional, labeling the construct as functional; and
in response to determining that the construct is non-functional, further training the deep machine learning model using the dataset, wherein each mapping in the dataset produces a functional construct.

9. The system of claim 8, wherein the indication of whether the construct is functional further indicates that a protein corresponding to the construct was determined to be secreted when expressed in a gram-positive host cell.

10. The system of claim 9, wherein the gram-positive host cell is a *Bacillus subtilis* cell.

11. The system of claim 7, wherein the deep machine learning model comprises an attention mechanism that incorporates a context of a respective amino acid in a given input sequence to generate an output sequence.

## Patentansprüche

1. Ein computerimplementiertes Verfahren zur Erzeugung einer Signalpeptid (SP)-Aminosäuresequenz, umfassend:
Trainieren eines tiefen maschinellen Lernmodells zur Erzeugung funktioneller SP-Sequenzen für Proteinsequenzen unter Verwendung eines Datensatzes, der eine Vielzahl von Ausgabe-SP-Sequenzen einer Vielzahl entsprechender Eingabe-Proteinsequenzen zuordnet;
Erzeugen, mittels des trainierten tiefen maschinellen Lernmodells, einer Ausgabe-SP-Sequenz für eine Eingabe-Proteinsequenz, wobei das trainierte tiefe maschinelle Lernmodell konfiguriert ist zum:
Empfangen der Eingabe-Proteinsequenz;
Tokenisieren jeder Aminosäure der Eingabe-Proteinsequenz zur Erzeugung einer Sequenz von Token;
Abbilden, mittels eines Encoders, der Sequenz von Token auf eine Sequenz kontinuierlicher Repräsentationen; und
Erzeugen, mittels eines Decoders, der Ausgabe-SP-Sequenz basierend auf der Sequenz kontinuierlicher Repräsentationen.

2. Das Verfahren nach Anspruch 1, ferner umfassend:
Erstellen eines Konstrukts durch Zusammenführen der erzeugten Ausgabe-SP-Sequenz und der Eingabe-Proteinsequenz; Bestimmen, ob das Konstrukt funktionell ist, durch Überprüfen, ob ein der Eingabe-Proteinsequenz entsprechendes Protein
(1) extrazellulär lokalisiert ist und
(2) eine native dreidimensionale Struktur annimmt, die biologisch funktionell ist,
wenn ein dem Ausgabe-SP entsprechendes Signalpeptid als Aminoterminus des Proteins dient.

3. Das Verfahren nach Anspruch 2, ferner umfassend:
als Reaktion auf die Feststellung, dass das Konstrukt funktionell ist, Kennzeichnen des Konstrukts als funktionell; und
als Reaktion auf die Feststellung, dass das Konstrukt nicht funktionell ist, weiteres Trainieren des tiefen maschinellen Lernmodells unter Verwendung des Datensatzes, wobei jede Zuordnung im Datensatz ein funktionelles Konstrukt erzeugt.

4. Das Verfahren nach Anspruch 2, wobei der Überprüfungsschritt das Exprimieren eines Proteins mit der Sequenz des Konstrukts in einer grampositiven Wirtszelle und das Nachweisen, ob das Protein sezerniert wird, umfasst.

5. Das Verfahren nach Anspruch 4, wobei die grampositive Wirtszelle eine Bacillus subtilis-Zelle ist.

6. Das Verfahren nach Anspruch 1, wobei das tiefe maschinelle Lernmodell einen Aufmerksamkeitsmechanismus umfasst, der einen Kontext einer jeweiligen Aminosäure in einer gegebenen Eingabesequenz einbezieht, um eine Ausgabesequenz zu erzeugen.

7. Ein System zur Erzeugung einer Signalpeptid (SP)-Aminosäuresequenz, umfassend:
einen Hardware-Prozessor, der konfiguriert ist zum:
Trainieren eines tiefen maschinellen Lernmodells zur Erzeugung funktioneller SP-Sequenzen für Proteinsequenzen unter Verwendung eines Datensatzes, der eine Vielzahl von Ausgabe-SP-Sequenzen einer Vielzahl entsprechender Eingabe-Proteinsequenzen zuordnet;
Erzeugen, mittels des trainierten tiefen maschinellen Lernmodells, einer Ausgabe-SP-Sequenz für eine Eingabe-Proteinsequenz, wobei das trainierte tiefe maschinelle Lernmodell konfiguriert ist zum:
Empfangen der Eingabe-Proteinsequenz;
Tokenisieren jeder Aminosäure der Eingabe-Proteinsequenz zur Erzeugung einer Sequenz von Token;
Abbilden, mittels eines Encoders, der Sequenz von Token auf eine Sequenz kontinuierlicher Repräsentationen; und
Erzeugen, mittels eines Decoders, der Ausgabe-SP-Sequenz basierend auf der Sequenz kontinuierlicher Repräsentationen.

8. Das System nach Anspruch 7, wobei der Hardware-Prozessor ferner konfiguriert ist zum:
Erstellen eines Konstrukts durch Zusammenführen der erzeugten Ausgabe-SP-Sequenz und der Eingabe-Proteinsequenz;
Empfangen einer Angabe, ob das Konstrukt funktionell ist;
als Reaktion auf die Feststellung, dass das Konstrukt funktionell ist, Kennzeichnen des Konstrukts als funktionell; und als Reaktion auf die Feststellung, dass das Konstrukt nicht funktionell ist, weiteres Trainieren des tiefen maschinellen Lernmodells unter Verwendung des Datensatzes, wobei jede Zuordnung im Datensatz ein funktionelles Konstrukt erzeugt.

9. Das System nach Anspruch 8, wobei die Angabe, ob das Konstrukt funktionell ist, ferner anzeigt, dass ein dem Konstrukt entsprechendes Protein als sezerniert bestimmt wurde, wenn es in einer grampositiven Wirtszelle exprimiert wurde.

10. Das System nach Anspruch 9, wobei die grampositive Wirtszelle eine Bacillus subtilis-Zelle ist.

11. Das System nach Anspruch 7, wobei das tiefe maschinelle Lernmodell einen Aufmerksamkeitsmechanismus umfasst, der einen Kontext einer jeweiligen Aminosäure in einer gegebenen Eingabesequenz einbezieht, um eine Ausgabesequenz zu erzeugen.

## Revendications

1. Procédé mis en œuvre par ordinateur pour générer une séquence d'acides aminés de peptide signal (SP), comprenant :
l'entraînement d'un modèle d'apprentissage automatique profond pour générer des séquences SP fonctionnelles pour des séquences de protéines en utilisant un ensemble de données qui associe une pluralité de séquences SP de sortie à une pluralité de séquences de protéines d'entrée correspondantes ;
la génération, via le modèle d'apprentissage automatique profond entraîné, d'une séquence SP de sortie pour une séquence de protéine d'entrée, dans lequel le modèle d'apprentissage automatique profond entraîné est configuré pour :
recevoir la séquence de protéine d'entrée ;
tokeniser chaque acide aminé de la séquence de protéine d'entrée pour générer une séquence de tokens ;
associer, via un encodeur, la séquence de tokens à une séquence de représentations continues ; et
générer, via un décodeur, la séquence SP de sortie sur la base de la séquence de représentations continues.

2. Procédé selon la revendication 1, comprenant en outre :
la création d'un construit en fusionnant la séquence SP de sortie générée et la séquence de protéine d'entrée ; la détermination si le construit est fonctionnel en vérifiant si une protéine correspondant à la séquence de protéine d'entrée
(1) est localisée de manière extracellulaire et
(2) acquiert une structure tridimensionnelle native qui est biologiquement fonctionnelle,
lorsqu'un peptide signal correspondant à la séquence SP de sortie sert d'extrémité amino de la protéine.

3. Procédé selon la revendication 2, comprenant en outre :
en réponse à la détermination que le construit est fonctionnel, l'étiquetage du construit comme fonctionnel ; et
en réponse à la détermination que le construit est non fonctionnel, l'entraînement supplémentaire du modèle d'apprentissage automatique profond en utilisant l'ensemble de données, dans lequel chaque association dans l'ensemble de données produit un construit fonctionnel.

4. Procédé selon la revendication 2, dans lequel l'étape de vérification comprend l'expression d'une protéine ayant la séquence du construit dans une cellule hôte gram-positive et la détection si la protéine est sécrétée.

5. Procédé selon la revendication 4, dans lequel la cellule hôte gram-positive est une cellule de Bacillus subtilis.

6. Procédé selon la revendication 1, dans lequel le modèle d'apprentissage automatique profond comprend un mécanisme d'attention qui incorpore un contexte d'un acide aminé respectif dans une séquence d'entrée donnée pour générer une séquence de sortie.

7. Système pour générer une séquence d'acides aminés de peptide signal (SP), comprenant :
un processeur matériel configuré pour :
entraîner un modèle d'apprentissage automatique profond pour générer des séquences SP fonctionnelles pour des séquences de protéines en utilisant un ensemble de données qui associe une pluralité de séquences SP de sortie à une pluralité de séquences de protéines d'entrée correspondantes ;
générer, via le modèle d'apprentissage automatique profond entraîné, une séquence SP de sortie pour une séquence de protéine d'entrée, dans lequel le modèle d'apprentissage automatique profond entraîné est configuré pour :
recevoir la séquence de protéine d'entrée ;
tokeniser chaque acide aminé de la séquence de protéine d'entrée pour générer une séquence de tokens ;
associer, via un encodeur, la séquence de tokens à une séquence de représentations continues ; et
générer, via un décodeur, la séquence SP de sortie sur la base de la séquence de représentations continues.

8. Système selon la revendication 7, dans lequel le processeur matériel est en outre configuré pour :
créer un construit en fusionnant la séquence SP de sortie générée et la séquence de protéine d'entrée ;
recevoir une indication si le construit est fonctionnel ;
en réponse à la détermination que le construit est fonctionnel, étiqueter le construit comme fonctionnel ; et en réponse à la détermination que le construit est non fonctionnel, entraîner davantage le modèle d'apprentissage automatique profond en utilisant l'ensemble de données, dans lequel chaque association dans l'ensemble de données produit un construit fonctionnel.

9. Système selon la revendication 8, dans lequel l'indication si le construit est fonctionnel indique en outre qu'une protéine correspondant au construit a été déterminée comme étant sécrétée lorsqu'elle est exprimée dans une cellule hôte gram-positive.

10. Système selon la revendication 9, dans lequel la cellule hôte gram-positive est une cellule de Bacillus subtilis.

11. Système selon la revendication 7, dans lequel le modèle d'apprentissage automatique profond comprend un mécanisme d'attention qui incorpore un contexte d'un acide aminé respectif dans une séquence d'entrée donnée pour générer une séquence de sortie.
